# FASCICULE DE BREVET EUROPEEN

(11) **EP 1 405 634 B1**
(45) Date de publication et mention de la délivrance du brevet: **11.04.2007**
(21) Numéro de dépôt: 03292053.0
(22) Date de dépôt: 19.08.2003
(51) Int. Cl.: A61K 8/00, A61K 8/02, A61K 8/18, A61K 8/368

(54) **Lingette et ses utilisations dans le domaine cosmétique**
Reinigungsartikel sowie ihre kosmetische Verwendung
Cleansing article and cosmetic use thereof

(30) Priorité: 19.09.2002 FR 0211607
(43) Date de publication de la demande: 07.04.2004
(73) Titulaire: L'ORÉAL, 75008 Paris (FR)
(72) Inventeur: Simon, Pascal, 94400 Vitry sur Seine (FR); Roche, Anne-Clotilde, 75006 (FR)
(74) Mandataire: Rasson, Catherine

(56) Documents cités:
- WO-A-01/35924
- WO-A-99/13861

## Description

L'invention se rapporte à un article, plus particulièrement une lingette, comportant au moins un substrat insoluble dans l'eau et une composition liquide anhydre comportant au moins une huile, au moins un tensioactif émulsionnant soluble dans la phase huileuse et ayant un HLB allant de 8 à 14, et au moins un gélifiant aqueux, ainsi qu'aux utilisations dudit article dans le domaine cosmétique ou dermatologique, en particulier pour le soin, le nettoyage et/ou le démaquillage de la peau humaine, plus spécialement du visage, et des yeux.

Les lingettes cosmétiques sont généralement constituées d'un substrat en une matière d'origine naturelle ou synthétique, qui est de préférence un non tissé, mais qui peut être aussi une mousse ou un tissu, ledit substrat étant imprégné d'une composition adaptée au but recherché, par exemple le nettoyage ou le démaquillage de la peau, ou encore le soin de la peau. Ces lingettes sont utilisées couramment et sont appréciées pour leur coté pratique car elles sont jetables et sont imprégnées de la quantité nécessaire et suffisante de produit nettoyant ou traitant. L'utilisation de ces lingettes évitent la manipulation et le transport de flacons contenant les lotions ou laits.

Les lingettes imprégnées peuvent être humides ou sèches. Les lingettes sèches doivent être mouillées avant leur utilisation et peuvent par exemple être imprégnées d'une composition moussante qui génère de la mousse quand la lingette est mouillée, comme décrit par exemple dans le document US-A-4,303,543. Les lingettes humides peuvent être imprégnées d'une composition aqueuse telle qu'une lotion démaquillante ou qu'un lait démaquillant par exemple, et, elles sont directement appliquées sur le visage ou le corps. Elles peuvent également être imprégnées d'une composition anhydre comportant par exemple un mélange d'huiles et de tensioactifs, et, la lingette est alors soit directement utilisée sur le visage ou le corps, soit préalablement humidifiée par un peu d'eau afin d'émulsionner le mélange huiles / tensioactifs avant l'application sur la peau, comme décrit par exemple dans le document US-A-6,136,775.

L'imprégnation des substrats par la composition d'imprégnation peut se faire selon différentes techniques, telles que la pulvérisation ou le trempage. Toutefois, ces techniques ne peuvent être utilisées que si les compositions d'imprégnation sont suffisamment fluides et ont une viscosité proche de celle de l'eau. En effet, il n'est pas possible de mouiller correctement le substrat quand les compositions sont trop visqueuses ; le substrat est alors incorrectement imprégné et, de plus, il est alors difficile à le découper, le plier et l'ensacher. En outre, l'article obtenu est désagréable à utiliser car le produit d'imprégnation reste en surface du substrat ou ne l'imprègne pas de manière homogène, si bien que certaines zones de l'article contiennent trop de produit, et d'autres en sont dépourvues ou en contiennent trop peu.

Ainsi, les compositions aptes à imprégner les substrats d'articles et notamment de lingettes sont toujours fluides ; ce sont généralement des lotions aqueuses ou hydroalcooliques, ou des émulsions fluides huile-dans-eau (H/E) qui ne contiennent pas ou très peu de gélifiant et une très faible fraction huileuse dispersée, car l'augmentation de la fraction huileuse dispersée dans une émulsion H/E augmente la viscosité de la composition et rend ainsi difficile son utilisation pour la fabrication d'une lingette. Ainsi, les documents WO-A-99/13861 et WO-A-01/35924 décrivent des articles substantiellement anhydres, dont les compositions d'imprégnation contiennent très peu d'huiles. En outre, cette huile est ajoutée directement sur le tissu imprégné d'une composition de tensioactif moussant, ce qui peut entraîner un défaut d'homogénéité sur l'article.
Les lingettes comprenant une composition à base d'huiles peuvent être utilisées pour le soin de la peau (imprégnation d'huiles pour le corps) ou pour le démaquillage de la peau (imprégnation d'huiles démaquillantes), mais lors de l'application sur la peau, elles laissent un film gras que la lingette, elle-même imbibée d'huile, ne peut pas éliminer. Ceci est d'autant plus désagréable au niveau de l'oeil quand la lingette est utilisée pour le démaquillage des yeux. Par ailleurs, les lingettes contenant des huiles associées à des tensioactifs sont utilisées après humidification avec un peu d'eau, l'addition d'eau permettant d'obtenir une émulsion par dispersion du mélange d'huile et de tensioactif dans l'eau, mais l'émulsion ainsi obtenue reste très liquide, sans consistance et a tendance à couler hors du substrat.

Ainsi, les compositions d'imprégnation des lingettes utilisées jusqu'à présent manquent de consistance et ne permettent pas d'obtenir une texture crémeuse, critères importants pour atteindre un bon confort d'utilisation aussi bien dans le soin que dans le démaquillage de la peau. Or, le confort apporté par un produit cosmétique, lors de son utilisation et immédiatement après son utilisation, est tout aussi important que son efficacité. Le fait d'obtenir une crème et non un fluide apporte un confort sur la peau, très appréciable.

Ainsi, il subsiste le besoin de disposer d'un article (lingette ou compresse ou mousse) pouvant donner lors de l'application sur la peau, une composition épaisse et onctueuse, cet article étant à la fois facile à utiliser et confortable à l'application.

La demanderesse a trouvé de manière surprenante une composition substantiellement anhydre contenant une ou plusieurs huiles, un ou plusieurs tensioactifs émulsionnants solubilisés, et un ou plusieurs gélifiants aqueux dispersés, qui est suffisamment fluide pour pouvoir être imprégnée par les moyens conventionnels sur une lingette ou tout autre substrat absorbant suffisamment résistant pour ne pas se déliter lors de son utilisation sur la peau, et qui est capable de générer une texture crémeuse épaisse lors de l'humidification par un peu d'eau. Ainsi, à partir d'une composition anhydre, il a été trouvé un moyen d'obtenir une lingette générant une composition de texture épaisse analogue à celle d'une crème, composition qu'il aurait été impossible d'imprégner directement sur une lingette par les moyens conventionnels. La composition selon l'invention présente l'avantage d'être substantiellement anhydre bien que contenant des polymères hydrophiles, et d'être imprégnées sur le substrat en donnant directement un article sensiblement sec, alors que généralement les articles secs sont obtenus par imprégnation d'une composition aqueuse et séchage, comme décrit par exemple dans le document WO-A-99/13861.

La présente invention a donc pour objet un article comportant (A) un substrat insoluble dans l'eau, comprenant une ou plusieurs couches, et (B) une composition substantiellement anhydre, ajoutée ou imprégnée sur le substrat, comprenant au moins 10 % en poids d'une ou plusieurs huiles par rapport au poids total de la composition, au moins un tensioactif émulsionnant et au moins un agent gélifiant hydrophile.

L'article obtenu est sensiblement sec, c'est-à-dire qu'il contient généralement moins de 10 % en poids d'eau, de préférence moins de 5 % en poids d'eau par rapport au poids total de l'article.

On entend par "substantiellement anhydre" une composition ne contenant pas d'eau ou contenant moins de 10 % en poids d'eau, et de préférence moins de 5 % en poids d'eau par rapport au poids total de la composition. La quantité d'eau dans la composition peut donc aller de 0 à 10 % et de préférence de 0 à 5 % du poids total de la composition.

La composition substantiellement anhydre de l'invention est liquide, c'est-à-dire qu'elle présente généralement une viscosité inférieure à 150 mPa.s et plus préférentiellement inférieure à 100 mPa.s. Cette viscosité va de préférence de 1 mPa.s à 100 mPa.s, mesurée à la température ambiante (environ 25°C) avec un appareil RHEOMAT RM 180, mobile 1 ou 2 selon la viscosité du liquide.

L'article selon l'invention présente l'avantage d'être très facile à manipuler, car il suffit de l'humidifier avec un peu d'eau, de le presser légèrement entre les doigts pour faire pénétrer l'eau et ainsi émulsionner la composition imprégnée avec l'eau. Il se forme alors une composition de texture crémeuse très agréable quant à son aspect, à son toucher, et aussi lors de son application sur la peau. En outre, l'utilisatrice peut régler à volonté la viscosité de la crème en rajoutant plus ou moins d'eau dans le substrat imprégné.

L'article selon l'invention est en particulier un article cosmétique, approprié pour le soin et/ou le traitement de la peau et pour le nettoyage ou le démaquillage de la peau du visage et/ou du corps et/ou des yeux. Il peut notamment constituer une lingette, mais il peut aussi se trouver sous forme d'un gant, d'une moufle ou sous toute autre forme appropriée pour une utilisation pratique sur le visage ou le corps.

L'invention a aussi pour objet l'utilisation cosmétique de l'article tel que défini ci-dessus, pour le soin, le nettoyage et/ou le démaquillage de la peau et/ou des yeux.

La composition utilisée selon l'invention pour l'imprégnation du substrat insoluble dans l'eau, étant destinée à une application topique, contient un milieu physiologiquement acceptable, c'est-à-dire compatible avec la peau, les muqueuses, les cheveux et le cuir chevelu.

Un autre avantage de l'article selon l'invention vient du fait qu'il ne contient pas d'eau ou qu'il en contient très peu. De ce fait, il n'est pas absolument nécessaire d'introduire des conservateurs pour protéger la formule. Ces lingettes peuvent donc être avantageusement exemptes de conservateurs, et utilisées plus particulièrement pour les peaux sensibles.

Aussi, l'invention a aussi pour objet l'utilisation cosmétique de l'article tel que défini ci-dessus, pour le soin, le nettoyage et/ou le démaquillage des peaux sensibles et/ou des yeux sensibles.

### I. Agents gélifiants hydrophiles

On entend par "agent hydrophile", un agent soluble ou dispersible dans l'eau.

On entend par "agent gélifiant", un agent qui augmente la viscosité d'une composition aqueuse le contenant.

La quantité d'agent gélifiant hydrophile dans la composition d'imprégnation de l'invention dépend de l'agent gélifiant utilisé et doit être telle que la composition à imprégner sur le substrat soit liquide et ait donc une viscosité inférieure à 150 mPa.s. Cette quantité peut aller par exemple de 0,1 à 20 % en poids (de matière active), de préférence de 0,5 à 15 % en poids, mieux de 1 à 10, % en poids, encore mieux de 1 à 6 % en poids et préférentiellement de 2 à 6 % en poids par rapport au poids total de la composition.

On peut utiliser tout type d'agent gélifiant hydrophile. Bien que tous les agents gélifiants hydrophiles permettent d'obtenir une texture crémeuse après humidification, on utilise de préférence un agent gélifiant capable d'épaissir quasi-instantanément et de manière homogène la composition après l'humidification de la lingette, ceci afin d'éviter à l'utilisatrice de devoir manipuler trop longtemps la lingette, et de devoir attendre trop longtemps que la lingette, après humidification, ne donne une composition crémeuse épaissie.

Selon un mode préféré de réalisation de l'invention, l'agent gélifiant hydrophile est un polymère hydrophile.

On peut utiliser en particulier les polymères se présentant sous forme de poudre ou sous forme d'émulsion eau-dans-huile (E/H) (émulsion inverse) contenant peu d'eau et telle que la composition finale contienne moins de 10 % d'eau et de préférence moins de 5 % d'eau. De manière préférée, on utilise les polymères se présentant sous forme d'émulsions E/H.

Par ailleurs, pour permettre une imprégnation homogène de la composition sur le substrat, il est préférable d'obtenir une dispersion du polymère dans l'huile relativement stable vis-à-vis de la sédimentation. Toutefois, si la dispersion de polymère dans l'huile n'est pas parfaitement stable, il est possible de réaliser la préparation de la composition et son imprégnation sur le substrat en continu, ou aussi d'agiter le récipient contenant la composition pour l'homogénéiser juste avant l'imprégnation du substrat.

Le polymère hydrophile utilisé comme agent gélifiant hydrophile est choisi de manière préférée parmi les polymères réticulés d'acide acrylique ou d'acide méthacrylique, les polymères d'acide 2-acrylamido 2-méthylpropane sulfonique, les copolymères réticulés d'acrylamide et d'acide 2-acrylamido 2-méthylpropane sulfonique, et leurs mélanges. Ces polymères sont capables d'épaissir quasi-instantanément et de manière homogène la composition après l'humidification de la lingette.

Ces homopolymères et copolymères réticulés sont réticulés par un agent de réticulation qui peut être notamment un composé à polyinsaturation oléfinique tel que ceux choisis dans le groupe comprenant le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'allypentaérythritol, l'arabitol, le mannitol, le sorbitol, l'allyle-sucrose ou le glucose. Préférentiellement, on utilise comme agent de réticulation, le méthylène bis-acrylamide. De préférence, l'agent de réticulation est présent dans le polymère ou le copolymère en une quantité allant de 0,06 à 1 millimole par mole de monomère ou du mélange de monomères.

Comme homopolymères réticulés d'acide acrylique ou d'acide méthacrylique, on peut citer par exemple ceux commercialisés sous les dénominations Carbopol 940, Carbopol 941, Carbopol 980, Carbopol 981, Carbopol ETD 2001, Carbopol ETD 2020, Carbopol ETD 2050, Carbopol 2984, Carbopol 5984, Carbopol Ultrez 10 par la société Goodrich, ceux commercialisés sous les dénominations Synthalen K, Synthalen L et Synthalen M par la société 3V ; ceux commercialisés sous les dénominations Modarez V1250 PX, Modarez V2000 PX, Viscaron A1600 PE, Viscaron A700 PE par la Société Protex.

Ces polymères étant anioniques, on ajoute de préférence à la composition un agent neutralisant. La quantité nécessaire d'agent neutralisant (base) est introduite dans la composition huileuse, de préférence sous la forme d'une base inorganique ou organique, telle que la soude, la potasse, l'ammoniaque ou une amine telle que la triéthanolamine ou la monoéthanolamine, ou leurs mélanges. De préférence, l'agent neutralisant est une amine liquide telle que la triéthanolamine par exemple qui se solubilise facilement dans la mélange huileux.

Les polymères d'acide 2-acrylamido 2-méthylpropane sulfonique peuvent être éventuellement réticulés par les agents de réticulation indiqués ci-dessus et/ou neutralisés par les bases décrites ci-dessus. Ils sont de préférence réticulés et au moins partiellement neutralisés. Comme polymères d'acide 2-acrylamido 2-méthylpropane sulfonique, on peut citer par exemple le poly(acide 2-acrylamido 2-méthylpropane sulfonique) commercialisé par la société Hoechst sous la dénomination commerciale « Hostacerin AMPS » (nom CTFA : ammonium polyacryldimethyltauramide).

Les copolymères réticulés d'acrylamide et d'acide 2-acrylamido 2-méthylpropane sulfonique (AMPS) préférés sont ceux obtenus par copolymérisation, par voie radicalaire, de 15-85 % en moles d'acrylamide et de 15-85 % en moles d'acide 2-acrylamido 2-méthylpropane sulfonique, notamment de 30-70 % en moles d'acrylamide et de 30-70 % en moles d'acide 2-acrylamido 2-méthylpropane sulfonique, et encore mieux de 55-70 % en moles d'acrylamide et de 30-45 % en moles d'acide 2-acrylamido 2-méthylpropane sulfonique.

Par ailleurs, l'acide 2-acrylamido 2-méthylpropane sulfonique peut être au moins partiellement neutralisé sous la forme d'un sel, par exemple par de la soude, par de la potasse, ou par une amine à faible poids moléculaire telle que la triéthanolamine ou la monoéthanolamine, ou leurs mélanges. De préférence, l'agent neutralisant est une amine liquide telle que la triéthanolamine par exemple qui se solubilise facilement dans la mélange huileux.

Selon un mode particulier de réalisation de l'invention, le copolymère anionique réticulé d'acrylamide et d'AMPS, utilisé dans la composition de l'invention se présente sous la forme d'une émulsion E/H. On peut citer par exemple l'émulsion E/H contenant environ 32 % d'eau, de 35 à 40 % en poids du copolymère, de 15 à 25 % en poids d'un mélange d'hydrocarbures isoparaffiniques en C₁₂-C_{13,} de 3 à 8 % en poids de tensioactif polyoxyéthyléné tel que le lauryléther de polyéthylèneglycol à 7 moles d'oxyde d'éthylène, émulsion commercialisée sous le nom de SEPIGEL 305 (nom C.T.F.A. : Polyacrylamide / C13-14 Isoparaffin /Laureth-7) par la société SEPPIC, et l'émulsion à 40 % de copolymère et 30 % d'eau, commercialisée sous le nom de SIMULGEL 600 (nom C.T.F.A. : Acrylamide / Sodium acryloyldimethyltaurate copolymer / Isohexadecane /Polysorbate 80) par la société SEPPIC.

Ces copolymères se présentant sous forme d'émulsion E/H présentent l'avantage de se disperser très bien dans le mélange d'huiles et de tensioactifs et de donner quasi instantanément une texture crémeuse et épaisse après humidification de la lingette. Par ailleurs, ces copolymères présentent l'avantage de permettre, après humidification de l'article selon l'invention, l'obtention d'une composition qui, selon la quantité plus ou moins importante d'eau introduite sur l'article, peut se présenter sous la forme d'une émulsion inverse eau dans l'huile (E/H), ou d'une émulsion directe huile dans l'eau (H/E) lorsque la quantité d'eau introduite est plus importante. L'obtention d'une émulsion E/H permet un démaquillage plus facile du fait que la phase huileuse est externe, la présence d'huiles augmentant l'efficacité du démaquillage des produits huileux présents sur la peau, notamment des produits de maquillage. Quand la quantité d'eau est plus importante et que l'on obtient une émulsion H/E, l'élimination du maquillage ou le rinçage de la peau sont facilités par la présence d'eau dans la phase externe de l'émulsion. Toutefois, comme indiqué ci-dessus, la composition selon l'invention comporte au plus 10 % en poids d'eau.

### II. Huiles

La composition contient au moins 10 % en poids d'une ou plusieurs huiles par rapport au poids total de la composition. Elle peut contenir une ou plusieurs huiles, notamment des huiles cosmétiques. La quantité d'huile(s) peut aller par exemple de 10 à 99 % en poids, de préférence de 30 à 90 % en poids, et mieux de 40 à 85 % en poids par rapport au poids total de la composition.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de coriandre, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12 ou le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1® " et "FLUTEC PC3® " par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050® " et "PF 5060® " par la Société 3M, ou encore le bromoperfluorooctyle vendu sous la dénomination "FORALKYL® " par la Société Atochem ; le nonafluorométhoxybutane vendu sous la dénomination "MSX 4518® " par la Société 3M et le nonafluoroéthoxyisobutane ; les dérivés de perfluoromorpholine, tels que la 4-trifluorométhyl perfluoromorpholine vendue sous la dénomination "PF 5052® " par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

### III. Tensioactifs

La composition contient un ou plusieurs tensioactifs émulsionnants. Ces tensioactifs permettent que, lors de l'humidification de l'article avec de l'eau, l'huile est émulsionnée avec l'eau en formant une crème. La quantité de tensioactif(s) peut aller par exemple de 0,1 à 90 % en poids, de préférence de 1 à 60 % en poids, mieux de 5 à 40 % en poids, et encore mieux 5 à 30 % en poids par rapport au poids total de la composition.

Le tensioactif ou le mélange de tensioactifs doit avoir un HLB (Hydrophilic Lipophilic Balance = Balance hydrophile lipophile) allant de 5 à 15, de préférence de 8 et 14, et il doit être soluble dans la phase huileuse.

Ces tensioactifs peuvent être non ioniques, anioniques, amphotères ou zwitterioniques.

De manière préférée, le ou les tensioactifs sont choisis parmi les tensioactifs non ioniques. Comme tensioactifs non ioniques, on peut citer par exemple les esters d'acide gras et de polyols, et leurs dérivés oxyalkylénés et notamment oxyéthylénés ; les éthers d'alcools gras et de polyols, et leurs dérivés oxyalkylénés et notamment oxyéthylénés, et leurs mélanges. Quand il s'agit d'esters d'acide gras et de polyols oxyalkylénés ou d'éthers d'alcools gras et de polyols oxyalkylénés, il peut y avoir par exemple de 1 à 150 groupes oxyalkylénés et notamment oxyéthylénés, et de préférence de 2 à 100 groupes oxyalkylénés et notamment oxyéthylénés.

Comme tensioactifs de ce type, on peut citer plus particulièrement :
- les esters d'acide gras et de sorbitan oxyéthylénés ou non oxyéthylénés, de préférence oxyéthylénés tels que (nom CTFA) Polysorbate 65, Polysorbate 85, PEG-5 Sorbitan lsostearate, PEG-20 Sorbitan Triisostearate, PEG-20 Sorbitan Isostearate, PEG-40 Sorbitan Septaoleate, PEG-20 Sorbitan Tetraoleate, PEG-20 Sorbitan Trioleate ;
- les esters d'acide gras et de glyceryle oxyéthylénés ou non, de préférence oxyéthylénés tels que (nom CTFA) PEG-20 glyceryl triisostearate, PEG-7 Glyceryl cocoate ;
- les esters d'acide gras et de polyglycéryle tels que (nom CTFA) Polyglyceryl-3 Triisostearate, Polyglyceryl-10 Diisostearate, Polyglyceryl-6 Isostearate, Polyglyceryl-3 Diisostearate, Polyglyceryl-10 Trioleate, Polyglyceryl-10 Trilaurate ;
- les esters d'acide gras et de polyéthylène glycol tels que (nom CTFA) PEG-8 Stearate, PEG-6 Oleate, PEG-6 Isostearate, PEG-12 Isostearate, PEG-12 Diisostearate, PEG-8 Isostearate, PEG-8 Diisostearate, PEG-10 Isostearate ;
- les éthers d'alcools gras polyoxyéthylénés et/ou polyoxypropylénés, comme par exemple le ceteareth-12 et le ceteareth-20 (nom CTFA), ainsi que les mélanges les contenant comme le mélange commercialisé sous la dénomination Emulgade CM par la société Henkel (mélange de isononanoate de cétéaryle, ceteareth-20, alcool cétéarylique, stearate de glyceryle, glycérine, ceteareth-12 et palmitate de cétyle)
- et leurs mélanges.

On peut aussi utiliser des tensioactifs ayant un HLB supérieur à 15, du moment qu'on y ajoute un ou plusieurs autres tensioactifs.

On peut ajouter aussi des tensioactifs moussants, notamment pour les articles, en particulier lingettes, de nettoyage ou de démaquillage de la peau. Comme tensioactifs de ce type, on peut citer par exemple :
(1) parmi les tensioactifs non ioniques, les polymères blocs oxyéthylénés oxypropylénés tels que le Poloxamer 184 (nom CTFA) ; les alkylpolyglycosides et notamment les alkylpolyglucosides (APG) ayant un groupe alkyle comportant de 6 à 30 atomes de carbone (alkyl-C₆-C₃₀ polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel ;
(2) parmi les tensioactifs anioniques, les alkylsulfates, les alkyl éther sulfates et leurs sels, notamment leurs sels de sodium, comme le mélange de Sodium Laureth Sulfate / Magnesium Laureth Sulfate / Sodium Laureth-8 Sulfate /Magnesium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie ;
(3) parmi les tensioactifs amphotères ou zwitterioniques, les dérivés alkylamido alkylamines tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA : sodium cocampho-acetate) et le mélange d'éthanolamides d'acide de coco (nom CTFA : Cocamide DEA).

Selon un mode préféré de réalisation de l'invention, la composition imprégnée dans l'article de l'invention comprend (i) de 1 à 6 % en poids d'un ou plusieurs gélifiants hydrophiles choisis parmi les polymères réticulés d'acide acrylique ou d'acide méthacrylique, les polymères d'acide 2-acrylamido 2-méthylpropane sulfonique et les copolymères réticulés d'acrylamide et d'acide 2-acrylamido 2-méthylpropane sulfonique ; (ii) de 30 à 90 % en poids d'une ou plusieurs huiles et (iii) de 5 à 40 % en poids d'un ou plusieurs tensioactifs émulsionnants non ioniques choisis parmi les esters d'acide gras et de polyols, et leurs dérivés oxyalkylénés ; les éthers d'alcools gras et de polyols, et leurs dérivés oxyalkylénés.

### IV. Additifs

La composition utilisée pour imprégner le substrat peut comprendre en outre les adjuvants classiquement mis en oeuvre dans le domaine cosmétique ou dermatologique. Elle peut contenir en particulier au moins un adjuvant choisi parmi les solvants organiques, les adoucissants, les antioxydants, les chélateurs, les parfums, les filtres U.V., les matières colorantes, les actifs hydrophiles ou lipophiles, les gélifiants lipophiles, les conservateurs, les vésicules lipidiques qui peuvent encapsuler éventuellement un ou plusieurs actifs, ou tout autre ingrédient habituellement utilisé en cosmétique ou dermatologie, et leurs mélanges. Les quantités des différents constituants des compositions selon l'invention sont celles classiquement utilisées dans les domaines considérés. Bien entendu, ces adjuvants doivent être de nature et utilisés en quantité telle qu'ils ne perturbent pas la composition selon l'invention. La quantité de ces adjuvants peut aller par exemple de 0,01 à 30 % en poids par rapport au poids total de la composition.

Les adjuvants peuvent être notamment choisis parmi les actifs lipophiles ou hydrophiles.

Comme actifs, on peut citer par exemple, les actifs antiséborrhéiques qui permettent un nettoyage de l'excédent de sébum sur la peau, et les agents antimicrobiens qui éliminent de la peau les microorganismes qui y sont éventuellement présents, et les mélanges de ces actifs.

Comme actifs antiséborrhéiques, on peut citer par exemple le soufre et les dérivés soufrés, le peroxyde de benzoyle, les dérivés de zinc tels que le sulfate de zinc et l'oxyde de zinc, le chlorure d'aluminium, le disulfure de sélénium, les vitamines B et notamment le panthénol (vitamine B5) et la niacinamide (vitamine B6 ou PP), et leurs mélanges.

Comme antimicrobiens, on peut citer par exemple les actifs suivants : dérivés de β-lactam, dérivés de quinolone, ciprofloxacine, norfloxacine, tétracycline et ses sels (chlorhydrate), érythromycine et ses sels (de zinc, estolate, stéarate), amikacine et ses sels (sulfate), 2,4,4'-trichloro-2'-hydroxy diphenyl éther (triclosan), 3,4,4'-trichlorocarbanilide (triclocarban), phénoxyéthanol, phénoxypropanol, phénoxyisopropanol, doxycycline et ses sels (chlorhydrate), capréomycine et ses sels (sulfate), chlorhexidine et ses sels (gluconate, chlorhydrate), chlorotétracycline et ses sels (chlorhydrate), oxytétracycline et ses sels (chlorhydrate), clindamycine et ses sels (chlorhydrate), éthambutol et ses sels (chlorhydrate), hexamidine et ses sels (iséthionate), métronidazole et ses sels (chlorhydrate), pentamidine et ses sels (chlorhydrate), gentamicine et ses sels (sulfate), kanamycine et ses sels (sulfate), linéomycine et ses sels (chlorhydrate), méthacycline et ses sels (chlorhydrate), méthenamine et ses sels (hippurate, mandelate), minocycline et ses sels (chlorhydrate), néomycine et ses sels (sulfate), netilmicine et ses sels (sulfate), paromomycine et ses sels (sulfate), streptomycine et ses sels (sulfate), tobramycine et ses sels (sulfate), miconazole et ses sels (chlorhydrate), amanfadine et ses sels (sulfate, chlorhydrate), octopirox, parachlorometaxylenol, nystatine, tolnaftate, zinc pyrithione, clotrimazole, acide salicylique, acide n-octanoyl-5 salicylique (ou acide capryloyl-salicylique), peroxyde de benzoyle, acide 3-hydroxybenzoique, acide glycolique, acide lactique, acide 4-hydroxy-benzoique, acide acétylsalicylique, acide 2-hydroxybutanoique, acide 2-hydroxypentanoique, acide 2-hydroxyhexanoique, acide phytique , acide N-acetyl-L-cysteine, acide lipoique, acide azélaïque, acide arachidonique, ibuprofen, naproxen, hydrocortisone, acetominophen, resorcinol, chlorhydrate de lidocaine, sulfate de néocycine, octoxyglycérine, octanoylglycine (ou capryloylglycine), caprylylglycol (1,2-octanediol), acide 10-hydroxy-2-décanoique, et leurs mélanges.

On peut citer aussi comme actifs, sans que cette liste ne soit limitative, les α-hydroxyacides comme l'acide lactique, l'acide glycolique, l'acide citrique, et leurs dérivés ; les huiles essentielles ; les vitamines et en particulier le rétinol (vitamine A), l'acide ascorbique (vitamine C), le tocophérol (vitamine E), le panthénol (vitamine B5) et leurs dérivés (esters par exemple) ; les co-enzymes et en particulier le co-enzyme Q10 ou ubiquinone ; les enzymes telles que par exemple les lipases, les protéases, les phospholipases, les cellulases, les peroxydases notamment les lactoperoxydases, les catalases, les superoxyde dismutases et les extraits végétaux contenant les enzymes précitées ; les levures telles que les *Saccharomyces Cerevisiae* ; les stéroïdes ; les anti-oxydants et anti-radicaux libres ; les hydratants tels que les polyols (glycérine, sorbitol, sucres), les hydrolysats de protéine, l'urée et les mélanges en contenant ; les agents antiélastase et anticollagénase ; les extraits végétaux et notamment les extraits de plancton ; et leurs mélanges.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-kéto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les adjuvants lipophiles sont dissous directement dans les huiles, tandis que les adjuvants hydrophiles sont dispersés dans la composition à l'aide des tensioactifs présents.

De manière préférée, la composition selon l'invention contient au moins un polyol tel que la glycérine, la quantité de polyol pouvant aller par exemple de 0,5 à 10 % en poids et mieux de 2 à 10 % en poids par rapport au poids total de la composition.

On peut ajouter aussi à la composition selon l'invention, un agent gélifiant lipophile à condition qu'il n'épaississe pas la composition d'imprégnation avant imprégnation sur le substrat. L'introduction d'un tel gélifiant permet d'obtenir un toucher filmogène quand l'article humidifié est appliqué sur la peau. Comme gélifiants lipophiles, on peut citer par exemple le palmitate de dextrine commercialisé sous la dénomination RHEOPEARL TL par la société Chiba Flour Milling.

Les compositions selon l'invention sont préparées selon le procédé suivant : On commence par préparer le mélange d'huiles, puis on y incorpore les tensioactifs à température ambiante ou à chaud selon qu'ils se présentent sous forme liquide ou solide, et on incorpore ensuite dans le mélange obtenu, l'agent gélifiant hydrophile et les adjuvants.

### Substrat

Le substrat insoluble dans l'eau peut comprendre une ou plusieurs couches et il peut être choisi dans le groupe comprenant des matériaux tissés, des matériaux non-tissés ; des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé épais, qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, les non tissés flockés commercialisés sous les dénominations Univel 109 et Univel 119 par la société Uni Flockage.

Ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

En outre, quand l'article est utilisé pour le corps, le substrat peut comprendre au moins une face rugueuse pour permettre en même temps le massage de la peau.

Par ailleurs, le substrat peut de manière avantageuse avoir aussi une capacité d'absorption d'eau suffisante pour sécher le corps, cette capacité d'absorption allant de préférence comprise de 800% à 3000%. Dans ce cas-là, les non-tissés utilisés sont de préférence de grammage élevé (grammage > 90 g/m²) et de forte épaisseur, ou bien ils contiennent au moins un polymère super-absorbant. Comme non-tissés de grammage élevé, on peut citer par exemple ceux commercialisés sous la dénomination Aquadim V100 par la société Tharreau, celui commercialisé sous la dénomination Norafin 1.111.00.01 par la société Jacob Holm, celui commercialisé sous la dénomination 112/132/4 par la société Freudenberg. Comme non tissés contenant un polymère super-absorbant, on peut citer ceux commercialisés sous les dénominations Dritex 120NN42 et 130WNNF60 par la société Georgia Pacific, et ceux commercialisés sous les dénominations HY0101046 ou HY0301038 par la société BBA.

Le substrat peut avoir toute taille et toute forme appropriées au but recherché. Il peut ainsi avoir par exemple la forme d'une lingette rectangulaire, ou la forme d'un gant ou d'une moufle, faciles à enfiler sur la main, ou une forme d'une compresse ronde. Il a généralement une surface comprise entre 0,005 m² et 0,1 m², de préférence entre 0,01 m² et 0,05 m².

Le taux d'imprégnation de la composition sur le substrat va généralement de 10 à 1500 %, de préférence de 50 à 500 % et encore mieux entre 70 et 250 %. Les techniques d'imprégnation des substrats par des compositions sont bien connues dans ce domaine et sont toutes applicables à la présente invention. En général, la composition d'imprégnation est ajoutée au substrat par une ou plusieurs techniques comprenant l'immersion, l'enduction, la vaporisation, etc.

L'invention a aussi pour objet un procédé cosmétique de soin, de nettoyage et/ou de démaquillage de la peau et/ou des yeux, consistant à passer sur la peau et/ou les yeux, un article tel que défini ci-dessus.

### Exemples de composition

Les exemples ci-après de compositions selon l'invention sont donnés à titre d'illustration et sans caractère limitatif. Les noms sont en nom chimique ou en nom CTFA. Les quantités y sont données en % en poids, sauf mention contraire.

### Exemple 1 : Compresse démaquillante

| **Composés** | **Quantités en %** |
|---|---|
| **Huile** | |
| Ethylhexyl palmitate | 76,5 |
| | |

| **Tensioactifs** | |
|---|---|
| PEG-20 glyceryl triisostearate | 8,5 |
| PEG-40 stearate | 2 |
| | |
| *Actif hydratant* | |
| Glycérine | 5 |
| | |

| **Epaississant hydrophile** | |
|---|---|
| Simulgel 600 (à 40 % en poids de polymère) (nom CTFA : Acrylamide / Sodium acryloyldimethyltaurate copolymer / Isohexadecane / Polysorbate 80) | 8 (soit 3,2 % de polymère) |

La composition de l'exemple 1 est imprégnée sur une compresse en non-tissé ronde, ovale, rectangulaire ou carrée dont la surface est adaptée pour le démaquillage du visage, par exemple 0,0016 m² à 0,01 m². Le taux d'imprégnation est de 100%.

Mode d'utilisation : Lors de l'utilisation, on prend la compresse dans la main, on la passe brièvement sous l'eau du robinet, puis on la passe sur le visage pour se démaquiller. En frottant la compresse sur le visage, la composition anhydre s'émulsionne instantanément avec l'eau rajoutée, pour former un lait démaquillant épais et onctueux très confortable et ne coulant pas. On peut ensuite rincer le visage à l'eau, passer une lotion tonique, ou laisser sécher.

### Exemple 2 : Lingette de soin ou gant de soin pour le corps

| **Composés** | **Quantités en %** |
|---|---|
| **Huiles** | |
| Ethylhexyl palmitate | 50 |
| Huile de Parleam | 25 |
| | |

| **Tensioactifs** | |
|---|---|
| PEG-20 glyceryl tri-isostéarate | 13 |
| PEG-40 stearate | 2 |
| | |
| *Actifs* | |
| Glycérine | 5 |
| | |

| **Epaississant hydrophile** | |
|---|---|
| Simulgel 600 (à 40 % en poids de polymère) (nom CTFA : Acrylamide / Sodium acryloyldimethyltaurate copolymer / Isohexadecane / Polysorbate 80) | 5 (soit 2 % de polymère) |

La composition est imprégnée sur une lingette dont la surface est adaptée pour une application sur toute la surface du corps, par exemple 0,02 m² à 0,25 m². Le taux d'imprégnation est de 200%.

De préférence, le substrat utilisé pour cet exemple a la forme d'un gant ou d'une moufle, ces formes présentant l'avantage de ne pas glisser sur le corps comme peut le faire une lingette. Le gant est de préférence en non tissé et il est constitué par exemple de deux feuilles de non-tissé découpées à la forme désirée et soudées, collés ou cousues à la périphérie. Le mode d'utilisation de ces gants ou moufles est simple : Après la douche ou le bain, le gant est passé sur la peau mouillée, sur l'ensemble du corps. Le passage du gant sur la peau mouillée émulsionne l'huile contenue dans le gant et la dépose sous la forme d'un lait corps hydratant onctueux et doux.

De manière avantageuse, le gant est constitué d'un non-tissé très absorbant, et il présente alors en outre l'avantage de permettre en même temps le séchage de la peau.

### Exemple 3 : Compresse démaquillante

| **Composés** | **Quantités en %** |
|---|---|
| ***Huiles*** | |
| Huile de Parleam | 40 |
| lsododécane | 28 |
| | |

| **Epaississant d'huile** | |
|---|---|
| Palmitate de dextrine (Rheopearl TL) | 2 |
| | |

| **Tensioactif** | |
|---|---|
| PEG-20 glyceryl tri-isostearate | 25 |
| | |

| **Epaississant hydrophile** | |
|---|---|
| Simulgel 600 (à 40 % en poids de polymère) (nom CTFA : Acrylamide / Sodium acryloyldimethyltaurate copolymer / Isohexadecane / Polysorbate 80) | 5 (soit 2 % de polymère) |

Le procédé d'imprégnation, le mode d'utilisation et les substrats sont identiques à ceux de l'exemple 1. Le taux d'imprégnation est de 150 %.

Cette compresse génère une émulsion inverse eau-dans-l'huile après humidification.

## Revendications

1. Article comportant (A) un substrat insoluble dans l'eau, comprenant une ou plusieurs couches, et (B) une composition substantiellement anhydre, ajoutée ou imprégnée sur le substrat, comprenant au moins 10 % en poids d'une ou plusieurs huiles par rapport au poids total de la composition, au moins un tensioactif émulsionnant soluble dans la phase huileuse et ayant un HLB allant de 8 à 14, et au moins un agent gélifiant hydrophile.

2. Article selon la revendication 1, **caractérisé en ce que** la composition comprend moins de 10 % en poids d'eau par rapport au poids total de la composition.

3. Article selon la revendication 1 ou 2, **caractérisé en ce que** l'agent gélifiant hydrophile est choisi parmi les polymères hydrophiles.

4. Article selon la revendication précédente, **caractérisé en ce que** le polymère hydrophile se présente sous forme de poudre ou sous forme d'émulsion eau-dans-huile.

5. Article selon la revendication 3 ou 4, **caractérisé en ce que** le polymère hydrophile est choisi parmi les polymères réticulés d'acide acrylique ou d'acide méthacrylique, les polymères d'acide 2-acrylamido 2-méthylpropane sulfonique, les copolymères réticulés d'acrylamide et d'acide 2-acrylamido 2-méthylpropane sulfonique, et leurs mélanges.

6. Article selon l'une quelconque des revendications précédentes, **caractérisé en ce que** l'agent gélifiant hydrophile est présent en une quantité de 0,1 à 20 % en poids, de préférence de 1 à 15 % en poids, par rapport au poids total de la composition.

7. Article selon l'une quelconque des revendications précédentes, **caractérisé en ce que** la composition comprend une quantité d'huile(s) allant de 10 à 99 % en poids, de préférence de 30 à 90 % en poids, par rapport au poids total de la composition.

8. Article selon l'une quelconque des revendications précédentes, **caractérisée en ce que** la composition comprend une quantité de tensioactif(s) allant de 0,1 à 90 % en poids, de préférence de 1 à 60 % en poids, par rapport au poids total de la composition.

9. Article selon l'une quelconque des revendications précédentes, **caractérisée en ce que** le tensioactif est un tensioactif non ionique choisi parmi les esters d'acide gras et de polyols, et leurs dérivés oxyalkylénés ; les éthers d'alcools gras et de polyols, et leurs dérivés oxyalkylénés, et leurs mélanges.

10. Article selon la revendication précédente, **caractérisé en ce que** le tensioactif non ionique est choisi parmi les esters d'acide gras et de sorbitan oxyéthylénés, les esters d'acide gras et de glycéryle oxyéthylénés, les esters d'acide gras et de polyglycéryle, les esters d'acide gras et de polyéthylène glycol, les éthers d'alcools gras polyoxyéthylénés et/ou polyoxypropylénés, et leurs mélanges.

11. Article selon l'une quelconque des revendications précédentes, **caractérisé en ce que** la composition comprend (i) de 1 à 6 % en poids d'un ou plusieurs gélifiants hydrophiles choisis parmi les polymères réticulés d'acide acrylique ou d'acide méthacrylique, les polymères d'acide 2-acrylamido 2-méthylpropane sulfonique et les copolymères réticulés d'acrylamide et d'acide 2-acrylamido 2-méthylpropane sulfonique ; (ii) de 30 à 90 % en poids d'une ou plusieurs huiles et (iii) de 5 à 40 % en poids d'un ou plusieurs tensioactifs émulsionnants non ioniques choisis parmi les esters d'acide gras et de polyols, et leurs dérivés oxyalkylénés ; les éthers d'alcools gras et de polyols, et leurs dérivés oxyalkylénés.

12. Article selon l'une quelconque des revendications précédentes, **caractérisé en ce que** la composition contient au moins un adjuvant choisi parmi les solvants organiques, les adoucissants, les antioxydants, les chélateurs, les parfums, les filtres, les matières colorantes, les actifs hydrophiles ou lipophiles, les gélifiants lipophiles, les vésicules lipidiques.

13. Article selon la revendication précédente, **caractérisé en ce que** l'adjuvant est un gélifiant lipophile.

14. Article selon l'une quelconque des revendications précédentes, **caractérisé en ce que** la composition présente une viscosité inférieure à 150 mPa.s.

15. Article selon l'une quelconque des revendications précédentes, **caractérisé en ce que** le substrat est choisi dans le groupe comprenant les matériaux tissés, les matériaux non-tissés, les mousses, les éponges, les ouates.

16. Article selon l'une quelconque des revendications précédentes, **caractérisé en ce que** le substrat est un non tissé à base de fibres d'origine naturelle ou d'origine synthétique.

17. Article selon l'une quelconque des revendications précédentes, **caractérisé en ce qu**'il comprend de 10 à 1500 % en poids de composition par rapport au poids de substrat.

18. Article selon l'une quelconque des revendications précédentes, **caractérisé en ce qu**'il se présente sous la forme de la forme d'une lingette rectangulaire, d'un gant, d'une moufle, ou d'une compresse ronde.

19. Article selon l'une quelconque des revendications précédentes, **caractérisé en ce qu**'il constitue un article de soin et/ou de traitement de la peau.

20. Article selon l'une quelconque des revendications 1 à 18, **caractérisé en ce qu'**il constitue un article de nettoyage et/ou de démaquillage de la peau et/ou des yeux.

21. Utilisation cosmétique de l'article selon l'une quelconque des revendications 1 à 18, pour le nettoyage et/ou le démaquillage de la peau et/ou des yeux.

22. Utilisation cosmétique de l'article selon l'une quelconque des revendications 1 à 18, pour le soin, le nettoyage et/ou le démaquillage des peaux sensibles et/ou des yeux sensibles.

23. Procédé cosmétique de soin, de nettoyage et/ou de démaquillage de la peau et/ou des yeux, consistant à passer sur la peau et/ou les yeux, un article selon l'une quelconque des revendications 1 à 18.

## Claims

1. Article containing (A) a water-insoluble substrate comprising one or more layers, and (B) a substantially anhydrous composition which is added to or impregnated into the substrate, comprising at least 10% by weight of one or more oils relative to the total weight of the composition, at least one emulsifying surfactant that is soluble in the oily phase and that has an HLB ranging from 8 to 14, and at least one hydrophilic gelling agent.

2. Article according to Claim 1, **characterized in that** the composition comprises less than 10% by weight of water relative to the total weight of the composition.

3. Article according to Claim 1 or 2, **characterized in that** the hydrophilic gelling agent is chosen from hydrophilic polymers.

4. Article according to the preceding claim, **characterized in that** the hydrophilic polymer exists in the form of a powder or in the form of a water-in-oil emulsion.

5. Article according to Claim 3 or 4, **characterized in that** the hydrophilic polymer is chosen from crosslinked polymers of acrylic acid or methacrylic acid, polymers of 2-acrylamido-2-methylpropanesulfonic acid, crosslinked copolymers of acrylamide and 2-acrylamido-2-methylpropanesulfonic acid, and mixtures thereof.

6. Article according to any one of the preceding claims, **characterized in that** the hydrophilic gelling agent is present in an amount of from 0.1% to 20% by weight and preferably from 1% to 15% by weight relative to the total weight of the composition.

7. Article according to any one of the preceding claims, **characterized in that** the composition comprises an amount of oil(s) ranging from 10% to 99% by weight and preferably from 30 to 90% by weight relative to the total weight of the composition.

8. Article according to any one of the preceding claims, **characterized in that** the composition comprises an amount of surfactant(s) ranging from 0.1% to 90% by weight and preferably from 1% to 60% by weight, relative to the total weight of the composition.

9. Article according to any one of the preceding claims, **characterized in that** the surfactant is a nonionic surfactant chosen from fatty acid esters of polyols and their oxyalkylenated derivatives; fatty alcohol ethers of polyols and their oxyalkylenated derivatives, and mixtures thereof.

10. Article according to the preceding claim, **characterized in that** the nonionic surfactant is chosen from oxyethylenated sorbitan fatty acid esters, oxyethylenated glyceryl fatty acid esters, polyglyceryl fatty acid esters, polyethylene glycol fatty acid esters and polyoxyethylenated and/or polyoxypropylenated fatty alcohol ethers, and mixtures thereof.

11. Article according to any one of the preceding claims, **characterized in that** the composition comprises (i) from 1% to 6% by weight of one or more hydrophilic gelling agents chosen from crosslinked polymers of acrylic acid or of methacrylic acid, 2-acrylamido-2-methylpropanesulfonic acid polymers and crosslinked copolymers of acrylamide and of 2-acrylamido-2-methylpropanesulfonic acid; (ii) from 30% to 90% by weight of one or more oils, and (iii) from 5% to 40% by weight of one or more nonionic emulsifying surfactants chosen from fatty acid esters of polyols, and oxyalkylenated derivatives thereof; fatty alkyl ethers of polyols, and oxyalkylenated derivatives thereof.

12. Article according to any one of the preceding claims, **characterized in that** the composition contains at least one adjuvant chosen from organic solvents, emollients, antioxidants, chelating agents, fragrances, screening agents, dyestuffs, hydrophilic or lipophilic active agents, lipophilic gelling agents and lipid vesicles.

13. Article according to the preceding claim, **characterized in that** the adjuvant is a lipophilic gelling agent.

14. Article according to any one of the preceding claims, **characterized in that** the composition has a viscosity of less than 150 mPa.s.

15. Article according to any one of the preceding claims, **characterized in that** the substrate is chosen from the group comprising woven materials, nonwoven materials, foams, sponges and cotton wool.

16. Article according to any one of the preceding claims, **characterized in that** the substrate is a nonwoven material based on fibres of natural origin or of synthetic origin.

17. Article according to any one of the preceding claims, **characterized in that** it comprises 10% to 1500% by weight of composition relative to the weight of the substrate.

18. Article according to any one of the preceding claims, **characterized in that** it is in the form of a rectangular wipe, a glove, a mitten or a round compress.

19. Article according to any one of the preceding claims, **characterized in that** it constitutes an article for caring for and/or treating the skin.

20. Article according to any one of Claims 1 to 18, **characterized in that** it constitutes an article for cleansing and/or removing makeup from the skin and/or the eyes.

21. Cosmetic use of the article according to any one of Claims 1 to 18, for cleansing and/or removing makeup from the skin and/or the eyes.

22. Cosmetic use of the article according to any one of Claims 1 to 18, for caring for, cleansing and/or removing makeup from sensitive skin and/or sensitive eyes.

23. Cosmetic method for caring for, cleansing and/or removing makeup from the skin and/or the eyes, which consists in passing over the skin and/or the eyes an article according to any one of Claims 1 to 18.

## Patentansprüche

1. Gegenstand umfassend (A) ein in Wasser unlösliches Substrat, das eine oder mehrere Schichten aufweist, und (B) eine im Wesentlichen wasserfreie Zusammensetzung, die zu dem Substrat gegeben wird oder mit der das Substrat getränkt wird und die mindestens 10 Gew.-% eines oder mehrerer Öle, bezogen auf das Gesamtgewicht der Zusammensetzung, mindestens einen in der Ölphase löslichen, emulgierenden grenzflächenaktiven Stoff mit einem HLB-Wert von 8 bis 14 und mindestens einen hydrophilen Gelbildner enthält.

2. Gegenstand nach Anspruch 1, **dadurch gekennzeichnet, dass** die Zusammensetzung weniger als 10 Gew.-% Wasser, bezogen auf das Gesamtgewicht der Zusammensetzung, enthält.

3. Gegenstand nach Anspruch 1 oder 2, **dadurch gekennzeichnet, dass** der hydrophile Gelbildner unter den hydrophilen Polymeren ausgewählt ist.

4. Gegenstand nach dem vorhergehenden Anspruch, **dadurch gekennzeichnet, dass** das hydrophile Polymer in Form von Pulver oder in Form einer Wasser-in-Öl-Emulsion vorliegt.

5. Gegenstand nach Anspruch 3 oder 4, **dadurch gekennzeichnet, dass** das hydrophile Polymer unter den vernetzten Polymeren von Acrylsäure oder Methacrylsäure, 2-Acrylamido-2-methyl-propansulfonsäurepolymeren, vernetzten Copolymeren von Acrylamid und 2-Acrylamido-2-methylpropansulfonsäure und deren Gemischen ausgewählt ist.

6. Gegenstand nach einem der vorhergehenden Ansprüche, **dadurch gekennzeichnet, dass** der hydrophile Gelbildner in einer Menge von 0,1 bis 20 Gew.-% und vorzugsweise 1 bis 15 Gew.-%, bezogen auf das Gesamtgewicht der Zusammensetzung, enthalten ist.

7. Gegenstand nach einem der vorhergehenden Ansprüche, **dadurch gekennzeichnet, dass** die Zusammensetzung einen Mengenanteil an Öl(en) von 10 bis 99 Gew.-% und vorzugsweise 30 bis 90 Gew.-%, bezogen auf das Gesamtgewicht der Zusammensetzung, enthält.

8. Gegenstand nach einem der vorhergehenden Ansprüche, **dadurch gekennzeichnet, dass** die Zusammensetzung einen Mengenanteil an einem oder mehreren grenzflächenaktiven Stoff(en) von 0,1 bis 90 Gew.-% und vorzugsweise 1 bis 60 Gew.-%, bezogen auf das Gesamtgewicht der Zusammensetzung, enthält.

9. Gegenstand nach einem der vorhergehenden Ansprüche, **dadurch gekennzeichnet, dass** es sich bei dem grenzflächenaktiven Stoff um einen nichtionischen grenzflächenaktiven Stoff handelt, der unter den Polyolfettsäureestern und deren alkoxylierten Derivaten; Polyolfettalkoholethern und deren alkoxylierten Derivaten und deren Gemischen ausgewählt ist.

10. Gegenstand nach dem vorhergehenden Anspruch, **dadurch gekennzeichnet, dass** der nichtionische grenzflächenaktive Stoff unter den ethoxylierten Sorbitanfettsäureestern, ethoxylierten Glycerylfettsäureestern, Polyglycerylfettsäureestern, Polyethylenglycolfettsäureestern, polyethoxylierten und/oder polypropoxylierten Fettalkoholethern und deren Gemischen ausgewählt ist.

11. Gegenstand nach einem der vorhergehenden Ansprüche, **dadurch gekennzeichnet, dass** die Zusammensetzung (i) 1 bis 6 Gew.-% eines oder mehrerer hydrophiler Gelbildner, die unter den vernetzten Acrylsäurepolymeren oder Methacrylsäurepolymeren, 2-Acrylamido-2-methylpropansulfonsäurepolymeren und vernetzen Copolymeren von Acrylamid und 2-Acrylamido-2-methylpropansulfonsäure ausgewählt sind; (ii) 30 bis 90 Gew.-% eines oder mehrerer Öle und (iii) 5 bis 40 Gew.-% eines oder mehrerer, nichtionischer, emulgierender grenzflächenaktiver Stoffe enthält, die unter den Polyolfettsäureestern und deren alkoxylierten Derivaten; Polyolfettalkoholethern und deren alkoxylierten Derivaten ausgewählt ist.

12. Gegenstand nach einem der vorhergehenden Ansprüche, **dadurch gekennzeichnet, dass** die Zusammensetzung mindestens einen Zusatzstoff enthält, der unter den organischen Lösungsmitteln, beruhigenden Stoffen, Antioxidantien, Chelatbildnern, Parfums, Filtern, Farbmitteln, hydrophilen oder lipophilen Wirkstoffen, lipophilen Gelbildnern und Lipidvesikeln ausgewählt ist.

13. Gegenstand nach dem vorhergehenden Anspruch, **dadurch gekennzeichnet, dass** der Zusatzstoff ein lipophiler Gelbildner ist.

14. Gegenstand nach einem der vorhergehenden Ansprüche, **dadurch gekennzeichnet, dass** die Zusammensetzung eine Viskosität unter 150 mPa·s besitzt.

15. Gegenstand nach einem der vorhergehenden Ansprüche, **dadurch gekennzeichnet, dass** das Substrat unter den Geweben, Nonwovens, Schäumen, Schwämmen und Watte ausgewählt ist.

16. Gegenstand nach einem der vorhergehenden Ansprüche, **dadurch gekennzeichnet, dass** das Substrat ein Nonwoven auf der Basis von Fasern natürlicher oder synthetischer Herkunft ist.

17. Gegenstand nach einem der vorhergehenden Ansprüche, **dadurch gekennzeichnet, dass** er 10 bis 1 500 Gew.-% der Zusammensetzung, bezogen auf das Gewicht des Substrats, enthält.

18. Gegenstand nach einem der vorhergehenden Ansprüche, **dadurch gekennzeichnet, dass** er in Form eines rechteckigen Tuchs, eines Fingerhandschuhs, eines Fäustlings oder einer runden Kompresse vorliegt.

19. Gegenstand nach einem der vorhergehenden Ansprüche, **dadurch gekennzeichnet, dass** es sich um einen Gegenstand für die Pflege und/oder Behandlung der Haut handelt.

20. Gegenstand nach einem der Ansprüche 1 bis 18, **dadurch gekennzeichnet, dass** er einen Gegenstand zur Reinigung und/oder zum Abschminken der Haut und/oder der Augen darstellt.

21. Kosmetische Verwendung des Gegenstands nach einem der Ansprüche 1 bis 18 für die Reinigung und/oder zum Abschminken der Haut und/ oder der Augen.

22. Kosmetische Verwendung des Gegenstands nach einem der Ansprüche 1 bis 18 für die Pflege, für die Reinigung und/oder zum Abschminken von empfindlicher Haut und/oder empfindlichen Augen.

23. Kosmetisches Verfahren zur Pflege, zur Reinigung und/oder zum Abschminken der Haut und/oder der Augen, das darin besteht, einen Gegenstand nach einem der Ansprüche 1 bis 18 über die Haut und/oder die Augen zu führen.
